(19) Europäisches Patentamt
European Patent Office
Office européen des brevets

(11) Publication number: **0 203 676 B1**

(12) **EUROPEAN PATENT SPECIFICATION**

(45) Date of publication of patent specification: **29.01.92**

(51) Int. Cl.[5]: **A61K 39/12**, A61K 37/02, A61K 39/245, A61K 39/385, C07K 17/00

(21) Application number: **86301223.3**

(22) Date of filing: **20.02.86**

A request for correction of the description and claims has been filed pursuant to Rule 88 EPC. A decision on the request will be taken during the proceedings before the Examining Division.

# (54) Vaccine for generating an immunogenic T cell response protective against a virus.

(30) Priority: **19.04.85 US 725087**

(43) Date of publication of application:
**03.12.86 Bulletin 86/49**

(45) Publication of the grant of the patent:
**29.01.92 Bulletin 92/05**

(84) Designated Contracting States:
**AT BE CH DE FR GB IT LI LU NL SE**

(56) References cited:
**WO-A-83/02897**

**Molecular immunology, vol. 21, 1984. T.P. Hopp "Immunogenicity of a synthetic HBsAg peptide : Enhancement by conjugation to a fatty acid carrier".Pages 13-16**

**Genetic variation amond influenza viruses, Academic Press, 1981, New York. L. Thibodeau et al "An influenza immunosome : its structure and antigenic properties. A model for a new type of vaccine" pages 587-600**

(73) Proprietor: **THE WISTAR INSTITUTE OF ANATOMY AND BIOLOGY**
**36th & Spruce Streets**
**Philadelpia, PA 19104(US)**

(72) Inventor: **Heber-Katz, Ellen**
**2300 Walnut Street**
**Philadelphia Pennsylvania 19103(US)**
Inventor: **Dietzschold, Bernhard**
**3430 Goshen Road**
**Newton Square, Pennsylvania 19073(US)**

(74) Representative: **Newby, John Ross et al**
**J.Y. & G.W. Johnson Furnival House 14/18**
**High Holborn**
**London WC1V 6DE(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to preparation of a vaccine for generating an immunogenic T cell response protective against a herpes virus.

Herpes viruses are widely spread in nature and natural hosts include foul and animals, including man. Man is the natural host for herpes simplex viruses types 1 and 2 (HSV-1 and HSV-2, respectively), varicella/zoster, cytomegalovirus (CMV) and Epstein-Barr virus (EBV). Herpes simplex viruses are known to cause many human diseases such as cold sores, encephalitis, eye infections, and genital infections, and HSV-2 has also been linked to cervical carcinoma. Clinical illness caused by herpes viruses presents a significent health problem and HSV-2 has produced major sociological consequences. At the present time no effective preventative measures are available.

There are experimental data, however, which indicate that immunization with a herpes virus or surface components of the virus can be protective. One such component, the virus envelope glycoprotein D molecule (gD), which is 59,000 MW, has been shown to be protective (Long, et al, Infect. and Immunity. 37: 761-764 (1984)). It has also been shown that antibody to this molecule can neutralize the virus and, when passively injected into animals, can be protective (Balanchandran et al, Infect. and Immunity, 37: 1132-1137 (1982); Dix et al., Infect. and Immun., 34: 192-199 (1981); Kapoor et al., J. Gen. Virol., 60: 225-233 (1982)). In all protocols for immunization against herpes virus thus published, high antibody titers have been reported. Therefore, it has been concluded generally that high antibody titers are extremely important in providing protection. Other studies indicate that such conclusion pertains to other virus infections as well.

It is known, however, that (1) recurrent herpes infections often occur in the presence of high antibody levels in the serum of patients and (2) individuals who have been infected previously with HSV-1 and are synthesizing antibody which crossreacts with both HSV-1 and HSV-2, nevertheless, contract HSV-2. More importantly, there have been studies showing not only the inability of antibody to protect, but that antibody can actually interfere with a protective host immune response (Wilson, et al., J. Immunology 132: 1522-1528 (1984); Babiuk et al., J. Microbiology, 25: 267 (1979)).

2. Objects of the Invention

It is an object of the invention to produce vaccines that offer significant protection for a long period of time against a large dose of a herpes virus.

It is a further object to provide vaccines which achieve such protection by a limited number of immunizations, in many instances a single immunization.

Another object of the invention is the development of an anti-HSV vaccine which utilizes a T cell response.

These and other objects of the invention will become further apparent from the detailed description of the invention, the appended claims, and the drawings in which

Figure 1 is a plot of the percentage of normal mice vs. time (days) comparing the protection against lethal infection by HSV-2 provided by the vaccine of the present invention as compared to certain control inoculants;

Figures 2a and 2b show the antibody binding activity induced by the vaccine of the invention in relation to certain control inoculants, and

Figure 3 is similar to Fig. 1, but using a reduced concentration of herpes virus ($4LD_{50}$ vs. $10LD_{50}$) in the challenge.

The vaccines of the present invention for generating a T cell response protective against a herpes simplex virus, type 1 or type 2, comprise an immunologically effective amount of (1) a peptide-fatty acid conjugate, the peptide having an amino acid sequence corresponding to the sequence of a fragment of herpes simplex virus type 1 glycoprotein gD-1 or herpes simplex virus type 2 envelope glycoprotein gD-2 or a synthetic replica of the said fragment, the conjugate having the formula

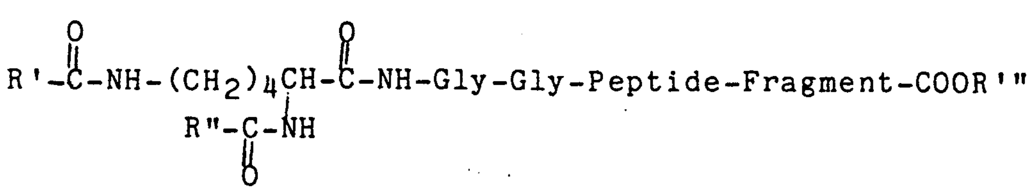

where R' and R'' are alkyl groups containing from 5 to 30, preferably from 10 to 20 carbon atoms, and R'''

is selected from the group consisting of hydrogen and at least one amino acid residue; (2) a liposome composition comprising a mixture of phosphatidyl choline, cholesterol and lysophosphatidyl choline, and (3) an adjuvant, whereby, said vaccine induces a T cell protective response in the substantial absence of an antibody response.

As used in the specification and appended claims, the expression "T cell response" refers to the ability of T cells to respond to antigen by production of lymphokines and/or molecules involved in effector functions, other than help for B cells in production of antibody, after antigen stimulation.

In a particularly preferred vaccine of the invention the Peptide Fragment in the Formula of the conjugate has the amino acid sequence

-Ser-Leu-Lys-Met-Ala-Asp-Pro-
Asn-Arg-Phe-Arg-
Gly-Lys-Asn-Leu-Pro- ,

R' and R'' are alkyl groups containing 15 carbon atoms, R''' is a cysteine residue and the adjuvant is alum.

Such peptide fatty acid conjugates may be prepared for example by adding a spacer of Gly-Gly-Lys to an N-terminal region of a virus glycoprotein which produces a T cell response and then adding two fatty acid side chains to the amines of the N-terminal lysine, the coupling of the fatty acid moieties being carried out by the symmetric anhydride method (Hopp, Molecular Immunology, 21: 13-16 (1984)).

According to a preferred form of the invention, the Gly-Gly-Lys spacer is added to an N-terminal region or fragment of the glycoprotein D molecule of HSV-1 or HSV-2 which produce a T cell response, following which a palmitic acid side chain is linked to each of the alpha and epsilon amino groups of the terminal lysine.

The N-terminal region of either gD-1 or gD-2 may comprise a synthetic peptide with sequence homology manually synthesized using Merrifield solid phase methods (Merrifield, J. Am. Chem. Soc. 85: 2149 (1963); Stewart et al, (ed), Solid phase peptide synthesis, W.H. Freeman and Co., San Francisco, CA (1969)).

The resulting products are useful as anti-virus vaccines, particularly anti-HSV-1 and anti-HSV-2 vaccines. The process is applicable to viruses generally and particularly to other members of the herpes virus group.

As stated, it was discovered that the vaccines of the invention achieve significant protection for a protracted period of time against a large dose of virus by a single injection. Considering the fact that prior researchers generally concluded that high antibody titers are extremely important in providing protection against HSV-1 and HSV-2 infection, it was surprising that a protective immune response against HSV infection need not stimulate an antibody response. Rather it was discovered that with vaccines of this invention a T cell proliferative response in the absence of an antibody response leads to effective long term protection.

The invention is described hereinafter in detail with respect to preparation and testing for immunogenicity of an anti-HSV-1 or HSV-2 vaccine comprising a synthetic peptide-fatty acid conjugate, the synthetic peptide having sequence homology with an N-terminal region or fragment of envelope glycoprotein D (gD) of either HSV-1 or HSV-2 which produces a T cell response.

Considering the constituent parts of the vaccine, the HSV specific component of the peptide-fatty acid conjugate comprises an N-terminal fragment of the surface glycoprotein D (gD), i.e. an amino acid peptide chain which produces a T cell response. The peptide may be the 23 amino acid peptide chain, the sequence of which was deduced from a terminal sequence of the gD molecule, a protein component of HSV-2 (Watson, Gene 158: 303 (1983)). The entire 23 amino acid sequence of the N-terminal fragment of HSV-2 gD is

(1) (2) (3) (4) (5) (6) (7)
$NH_2$-Lys-Tyr-Ala-Leu-Ala-Asp-Pro-

(8) (9)(10)(11)(12)(13)(14)(15)
Ser-Leu-Lys-Met-Ala-Asp-Pro-Asn-

(16)(17)(18)(19)(20)(21)(22)
Arg-Phe-Arg-Gly-Lys-Asn-Leu-

(23)
Pro-COOH

The 23 amino acid sequence of the N-terminal fragment of HSV-1 gD differs from that of HSV-2 gD in that alanine is at position 7 and aspartic acid is at position 21 (Dietzschold et al., J. of Viro., 52, No.2: 431-435 (1984)).

Rather than using the entire 23 amino acid N-termial protein of HSV-2 gD or HSV-1 gD, certain subunits thereof may be used provided they give a T cell response. A typical subunit of HSV-2 gD is

(8) (9)(10)(11)(12)(13)(14)
-Ser-Leu-Lys-Met-Ala-Asp-Pro-

(15)(16)(17)(18)
-Asn-Arg-Phe-Arg-

(19)(20)(21)(22)(23)
-Gly-Lys-Asn-Leu-Pro-

As previously stated the synthetic replicas of the above-noted N-terminal fragments may be prepared using solid phase methods (see Merrifield, J. Am. Chem. Soc. 85: 2149 (1963); Stewart et al (ed), Solid phase peptide synthesis, W.H. Freeman and Co., San Francisco (1969)).

There is added to the N-terminus of the peptide fragments a spacer of Gly-Gly-Lys$(NH_2)_2$. Such addition can be accomplished as part of the synthesis of the N-terminal peptide fragment. Preferably, cysteine is also added at the C-terminus for linkage to other "carrier" molecules, which are generally relatively large proteins.

There are then coupled to the alpha and epsilon amines of the lysine terminus saturated fatty acids generally having from about 11 to about 21 carbon atoms, examples of which are palmitic, stearic and oleic acids. The procedure by which conjugates of this general type are formed is well known (Hopp, Molecular Immunology. 21: 13-16 (1984)).

The peptide-fatty acid conjugate, prepared as above, is mixed with a liposome composition comprising a mixture of three lipids, namely phosphatidyl choline, cholesterol and lysophosphatidyl choline, following the method of Thibodeau (see Thibodeau et al, "Genetic variation among influenza viruses", Acad. Press, N.Y., London (1981) p 587). The weight proportion of the three constituents of which the liposome is formed may vary considerably. Preferably, the proportion by weight of the three lipids is 16:2:1, respectively.

The peptide-fatty acid conjugate-containing liposome preparation is then mixed with an adjuvant, e.g. alum or complete Freund's adjuvant. Generally a weight ratio of 1:1 is used when the adjuvant is CFA and results in an emulsion which is available for use as a vaccine. In the case of alum, from about 4 to about 16 parts, preferably about 8 parts by weight per part of peptide (in the absence of spacer and fatty acid) may generally be used.

The vaccine is administered in a dosage range of from about 100 to 300 $\mu$g, preferably about 150 $\mu$g, based on the weight of peptide per se (in the absence of spacer and fatty acid), in order to obtain the desired immunogenic T cell response protective against HSV-1 and HSV-2 virus. Usually the vaccine may be administered in a single dose and protection against a large dose of herpes virus is provided. However, a series of doses at intervals of several weeks or months followed, if necessary, by a booster dose at an interval of serval months to several years may be administered if necessary. So used, the vaccine will produce in laboratory animals a T cell response protective against a herpes virus.

The following examples further illustrate the present invention without, however, limiting the same

thereto.

Example 1 A peptide having the sequence homology of the 23 amino acid peptide chain, the sequence of which was deduced from a N-terminal gD molecule of HSV-2, having a spacer of Gly-Gly-Lys added to the N-terminus and cysteine added to the C-terminus, and having the following formula was prepared by Merrifield solid phase methods (Merrifield 1963; Stewart et al, 1969, supra)

$(NH_2)_2$Lys-Gly-Gly)*-Lys-Tyr-Ala-Leu-
Ala-Asp-Pro-Ser-Leu-Lys-Met-
Ala-Asp-Pro-Asn-Arg-Phe-Arg-
Gly-Lys-Asn-Leu-Pro-(Cys)**COOH

* A spacer group added to the peptide and to which palmitic acid side chains are added to the Lys amino groups.

** A cysteine amino acid used for linkage to other carrier molecules.

More specifically the peptide molecule having the amino acid sequence immediately above, and sometimes referred to herein as "1-23(2)", was synthesized as follows:

All N-tert-butoxycarbonyl (BOC) amino acids were purchased from Sigma Chemical Co., BOC-Cys-O-Resin, L-t-Amyloxycarbonyl-N-Tosyl-L-Arginine, and L-BOC-O-Benzyl-L-Serine were purchased from Peninsula Laboratories, Inc. Peptides were manually synthesized using Merrifield solid phase methods (Merrifield, 1963; Stewart, 1969, supra) with the following modifications.

(1) A series of three washes, the first with methylene chloride, the second with absolute ethanol and the third with methylene chloride was used instead of dioxane and chloroform before and after deprotection of N-t-BOC amino acid groups; (2) N-t-BOC amino acids were deprotected with 25% trifluoroacetic acid in methylene chloride; (3) completeness of the deprotection and coupling reactions was monitored using the color tests described by Kaiser et al., Annal. Biochemistry, 34: 595-598 (1970). After synthesis the resin was dried, and 50 equivalents of thioanisole were added. The side protection groups were removed and the peptide was cleaved from the resin with anyhydrous hydrogen fluoride. After removal of the anyhydrous hydrogen fluoride, the peptide resin mixture was extensively washed with ethyl acetate and ether to remove the thioanisole. The cleaved peptide was extracted with 1.5% $NH_4CO_3$ and lyophilized. The amino acid sequence of the peptide was verified by automated Edman degradation as described by Hunkapillar and Hood (Biochemistry 17: 2124-2133 (1978)).

Example 2

To add palmitic acid side chains, the N-terminal lysine was coupled as the bis-t-butyloxycarbonyl derivative, then deprotected using trifluoroacetic acid. The palmitic acid moieties were coupled by the symmetric anhydride method (Hopp, 1984, supra). Thus, the molecule is:

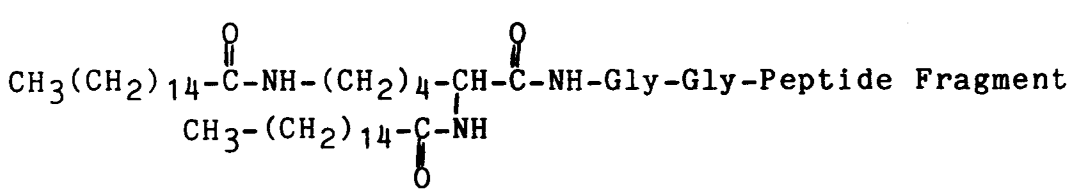

Example 3

The fatty acid-peptide conjugate of Example 2 was then mixed with a liposome comprising 3 lipids as follows (see Thibodeau et al, (1981)):

Phosphatidyl choline, cholesterol, and lysolecithin were each dissolved in MeOH/chloroform (1:3) and then mixed in the ratio of 16:2:1, respectively. This mixture was then blown down with $N_2$ rotating the vial in warm $H_2O$ to get an even film over the entire vial. Five mg. of the peptide-palmitic acid conjugate was dissolved in 2 ml of a 1% octylglycoside in phosphate buffered saline solution (PBS). Ten mg. of the lipid mixture was then added to this peptide solution. Dialysis was carried out against PBS using a 3500 dalton cutoff Spectropore dialysis mebrane for 24 hours. The liposomes were sonicated for 5 minutes.

The peptide-containing liposome preparation was then mixed with complete Fruend's adjuvant (CFA) in a ratio of 1:1 to form a vaccine emulsion.

Example 4

Testing of the vaccine.

The first experiment was done with a single injection of vaccine into the two hind footpads of a group of six Balb/c mice. The volume given was approximately 0.2 ml/animal or 10 µg/g body weight. In addition, as controls Balb/c mice (6 mice/group) were immunized with 1-23(2) peptide in CFA, CFA alone, and UV-inactivated HSV-1 in CFA, as follows:

(1) 1-23(2) at 100 µg/animal;
(2) UV-inactivated HSV-1 at 10 PFU/animal;
(3) Vaccine of Example 3 (1-23(2)-palmitic acid-liposome, CFA) at approximately 150 µg peptide/animal;
(4) CFA alone.

Six and 1/2 months after this single immunization, the mice were challenged with a lethal dose of HSV-2 (a 10 $LD_{50}$ dose of strain 186 grown in BHK cells which are mouse fibroblasts) in both hind footpads and the animals were examined for the next 30 days for paralysis and death. The results are plotted in Figure 1. It can be seen that by day 8, many of the animals were symptomatic. Only two groups, the HSV-1-primed (-♦-♦-) and the 1-23(2) palmitic acid-liposome-CFA-primed (-□-□-) animals, appeared normal. On day 18, when all of the control animals had died (CFA-◊-◊-; 1-23(2) (-■-■-), the HSV-1 and 1-23(2)-palmitic acid-liposome, CFA vaccine immunized animals survived. At day 120, 33% of both of those groups were normal.

Example 5

To determine the mechanism of protection, the antibody response (humoral response in terms of antibody which can bind the peptide to virus and which can neutralize the activity of virus infectivity in an in vitro assay) was studied. T cells responses, by measuring the ability of T cells to respond to antigen by their production of lymphokines after antigen simulation were also determined. First, since it is known that neutralizing antibody can protect animals from an HSV infection (Balachandran, 1982 supra; Dix, et al, Infec. and Immun., 34: 192-199 (1981) Kapoor et al, J. Gen. Virol., 60: 225-233 (1982)), an effort was made to determine if this was the cause of the protection seen.

Animals were bled 1 week after HSV-2 challenge since specific antibody which had already been induced by previous exposure to antigen (peptide) should at that time have been high, and serum was taken from animals and frozen until time of assay. The results obtained as seth forth in Table 1, below.

TABLE 1

Neutralization Titers of Anti-HSV Antibody

| Pooled Serum* From Each Group | Neutralizing Antibody for HSV-1 | HSV-2 |
|---|---|---|
| 1. Control | 12** | 6 |
| 2. HSV-1 | 389 | 97 |
| 3. 1-23(2) | 6 | 8 |
| 4. Vaccine of Example 3 (1-23(2)-liposome CFA) | 5 | 5 |

* Animals were challenged with HSV-2 in the foodpads 6 and 1/2 months after a single immunization of antigen in CFA. Bleedings were done 1 week after challenge with HSV-2. 100 PFU of virus in 25 microliters were added to serum antibody in the same volume with the serum being diluted in two fold dilutions. This mixture in 96 well Costar plates was incubated for 1 hour at 37°C and BHK cells were added at a concentration of 5 x $10^4$ cells/ml in 50 microliters. Three to four days later, the cells were stained with crystal violet dissolved in 10% buffered formalin.

** Titer=1/dilution of sample x $2^x$; x=well number

As shown in Table 1, neutralizing antibody titers could explain the protection in the HSV-1 group, but could not explain the protection seen with the group immunized with the vaccine of Example 3 (1-23(2)-liposome CFA).

Example 6

This example pertains to detection of anti-viral binding activity and should be considered in connection with Figures 2(a) and 2(b). Balb/c mice were challenged with HSV-2 in the hind footpads 6 and 1/2 months after single immunization with the vaccine of Example 3. Bleedings were done 1 week after challenge with HSV-2. A radioimmunoassay was carried out using HSV-1 (-○-○-) and HSV-2 (-△-△-) infected and uninfected (-■-■-) BHK lysates, and 1-23(H) peptides (-●-●-) and phosphate buffered saline alone (-▼-▼-) as immunoadsorbants. The assay was done by (a) preincubation of vinyl 96 well culture plates with the various immunoadsorbants in saline overnite at 4°C; (b) coating with 50% Fetal Calf Serum; (c) incubation of the Balb/c antisera for 3 hours at room temperature, and (d) labeling with 5000 CPM of $^{125}$I-rabbit Fab anti-mouse Ig. Figures 2(a) and 2(b) show the amount of radioactively labeled anti-mouse antibody bound versus the dilution of the antiserum tested. The highest Concentration shown is considered the nonspecific binding region. As shown by the figures, anti-peptide binding activity at the highest concentration tested could be detected when the animals have been immunized with 1-23(2)-lipsome (A) and 1-23-(2) (C), but not with HSV-1 (B) or CFA (D). The nature of the binding activity is unclear since it is detected in the non-specific part of the titration curve. However, it is clear that antibody to the virus was detected only in animals immunized with HSV-1 (B).

In view of these experiments it was concluded that the vaccine of the invention induces no detectable antibody which can bind the virus.

Example 7

T cells from animals immunized with the vaccine of Example 3 were obtained from the lymph nodes, purified on nylon wool columns (Julius, et al, Europ. J. Immunol. 3: 645 (1973)), and then tested for responsiveness in vitro by T cell proliferation measured through the incorporation of $^3$H-thymidine into DNA after three days in culture (Corradin, et al., J. Immunol. 119: 1048 (1977)). The results appear in Table 2.

TABLE 2

| | |
|---|---|
| T cells alone | 7,300 CPM |
| T cells + 1-23(2) peptide 50 μg/ml | 30,400 CPM |
| T cells + HSV-1 ($10^6$ PFU/ml) | 20,300 CPM |

The data in Table 2 show that T cells from animals vaccinated respond to the peptide and also cross-react with the virus. The T cell proliferation test in vitro has generally been accepted as indicative of the presence of an antigen specific T cell response as correlating with T cell effector function. T cells induced by the peptide-containing vaccine are responsive not only to the specific peptide but also to a corresponding exiologic agent of the herpes virus infection, i.e. HSV-1.

Example 8

A further experiment was carried out in the same manner as Example 4. Animals were immunized in the hind footpads with the following: (a) 1-23(2)-palmitic acid-liposomes in CFA (13 animals) (-□-□-); (b) UV-inactivated HSV-1 in CFA (5 animals) (-□-□-); (c) CFA alone (10 animals) (-◊-◊-); (d) liposomein in CFA (11 animals) (-♦-♦-); and (e) 1-23(2)-palmitic acid-liposome in saline (5 animals) (-■-■-). The animals were then challenged 3 and 1/2 months after immunization with a 4 LD 50 of HSV-2(186). The results are presented in Figure 3.

The published articles identified in the foregoing specification are incorporated by reference herein in their entirety.

**Claims**

**Claims for the following Contracting States : BE, FR, DE, IT, LU, NL, SE, CH, GB**

1. A vaccine for generating an immunogenic T cell response protective against a Herpes simplex virus, type 1 or type 2, said vaccine comprising an immunologically effective amount of (1) a peptide-fatty acid conjugate, said peptide having an amino acid sequence corresponding to the sequence of a fragment of herpes simplex virus type 1 envelope glycoprotein gD-1 or herpes simplex virus type 2 envelope glycoprotein gD-2 or a synthetic replica of said fragment, said conjugate having the formula

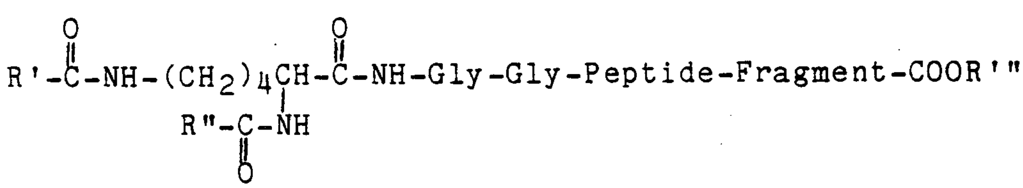

where $R^1$ and R'' are alkyl groups containing from 5 to 30 carbon atoms, and R''' is selected from the group consisting of hydrogen and at least one amino acid residue; (2) a liposome composition comprising a mixture of phosphatidyl choline, cholesterol and lysophosphatidyl choline, and (3) an adjuvant, whereby said vaccine induces a T cell protective response in the substantial absence of an antibody response.

2. A vaccine according to claim 1 in which each of R' and R'' is an alkyl group containing from 10 to 20 carbon atoms, R''' is a cysteine residue, and the adjuvant is selected from the group consisting of alum and complete Freund's adjuvant.

**3.** A vaccine according to claim 1 in which each of R' and R'' is an alkyl group containing 15 carbon atoms, R''' is a cysteine residue, and the adjuvant is alum.

**4.** A vaccine according to claim 3 in which said phosphatidyl choline, cholesterol and lysophosphatidyl choline are present in the proportions by weight of 16:2:1, respectively.

**5.** A vaccine according to any of claims 1 to 4 in which the Peptide Fragment in the formula of said conjugate is

-Ser-Leu-Lys-Met-Ala-Asp-Pro-
Asn-Arg-Phe-Arg-Gly-Lys-Asn-
Leu-Pro-.

**6.** A vaccine according to claim 3 or 4 in which the Peptide Fragment in the formula of said conjugate is

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro-.

**7.** A vaccine according to any of claims 1 to 4 in which the Peptide Fragment in the formula of said conjugate is

-Ser-Leu-Lys-Met-Ala-Asp-Pro -
Asn-Arg-Phe-Arg-Gly-Lys-
Asp-Leu-Pro-.

**8.** A vaccine according to any of claims 1 to 4 in which the Peptide Fragment in the formula of said conjugate is

-Lys-Tyr-Ala-Leu-Ala-Asp-Ala-
Ser-Leu-Lys-Met-Ala-Asp-Pro-Asn-
Arg-Phe-Arg-Gly-Lys-Asp-Leu-Pro-.

**9.** A peptide-fatty acid conjugate having the formula

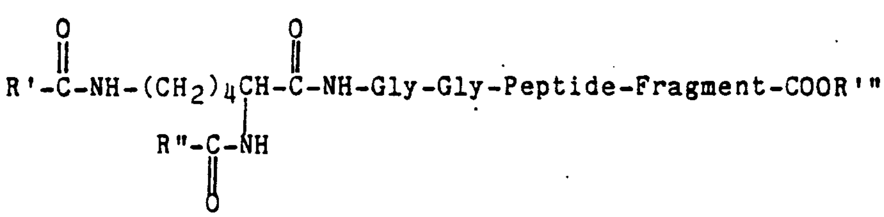

where R' and R'' are alkyl groups containing from 5 to 30 carbon atoms, R''' is selected from the group

consisting of hydrogen and at least one amino acid residue, and said peptide fragment has an amino acid sequence corresponding to the sequence of a fragment of herpes simplex virus type 1 envelope glycoprotein gD-1 or herpes simplex virus type 2 envelope glycoprotein gD-2 or a synthetic replica of said fragment, whereby said conjugate induces a T cell protective response in the substantial absence of an antibody response.

**10.** A conjugate according to claim 9 in which each of R' and R'' is an alkyl group containing from 10 to 20 carbon atoms, and R''' is a cysteine residue.

**11.** A conjugate according to claim 9 in which each of R' and R'' is an alkyl group containing 15 carbon atoms, and R''' is a cysteine residue.

**12.** A conjugate according to any of claims 9 to 11 in which the Peptide Fragment in the formula is

-Ser-Leu-Lys-Met-Ala-Asp-Pro-
Asn-Arg-Phe-Arg-Gly-Lys-Asn-
Leu-Pro- .

**13.** A conjugate according to claim 11 in which the Peptide Fragment in the formula is

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro- .

**14.** A conjugate according to any of claims 9 to 11 in which the Peptide Fragment in the formula is

-Ser-Leu-Lys-Met-Ala-Asp-Pro-
Asn-Arg-Phe-Arg-Gly-Lys-
Asp-Leu-Pro- .

**15.** A conjugate according to any of claims 9 to 11 in which the Peptide Fragment in the formula is

Lys-Tyr-Ala-Leu-Ala-Asp-Ala-
Ser-Leu-Lys-Met-Ala-Asp-Pro-Asn-
Arg-Phe-Arg-Gly-Lys-Asp-Leu-Pro- .

**16.** A composition useful for forming a vaccine for generating a T cell response protective against a herpes simplex virus, type 1 or type 2, when combined with an adjuvant, comprising an immunologically effective amount of (1) a peptide-fatty acid conjugate according to any of claims 9 to 15 in which said peptide fragment has an amino acid sequence corresponding to the sequence of a fragment of herpes simplex virus type 1 envelope glycoprotein gD-1 or herpes simplex virus type 2 envelope glycoprotein

gD-2 or a synthetic replica of said fragment, and (2) a liposome composition comprising a mixture of phosphatidyl choline, cholesterol and lysophosphatidyl choline, said composition being capable of inducing a T cell protective response in the substantial absence of an antibody response.

**17.** A composition according to claim 16 in which said phosphatidyl choline, cholesterol and lysophosphatidyl choline are present in the proportions by weight of 16:2:1, respectively.

**18.** A composition according to claim 16 or 17 in which the Peptide Fragment in the formula of said conjugate is

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro-.

**Claims for the following Contracting State : AT**

**1.** A vaccine for generating an immunogenic T cell response protective against a Herpes simplex virus, type 1 or type 2, said process comprising mixing of immunologically effective amounts of (1) a peptide-fatty acid conjugate, said peptide having an amino acid sequence corresponding to the sequence of a fragment of herpes simplex virus type 1 envelope glycoprotein gD-1 or herpes simplex virus type 2 envelope glycoprotein gD-2 or a synthetic replica of said fragment, said conjugate having the formula

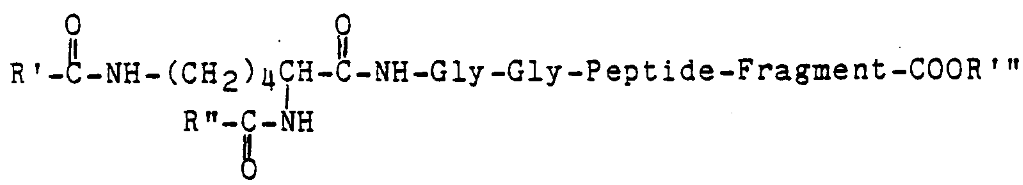

where R' and R" are alkyl groups containing from 5 to 30 carbon atoms, and R''' is selected from the group consisting of hydrogen and at least one amino acid residue; (2) a liposome composition comprising a mixture of phosphatidyl choline, cholesterol and lysophosphatidyl choline, and (3) an adjuvant, such that the vaccine prepared induces a T cell protective response in the substantial absence of an antibody response.

**2.** A process according to claim 1 in which for each of R' and R" in the conjugate an alkyl group containing from 10 to 20 carbon atoms, and for R''' a cysteine residue are used, and the adjuvant is selected from the group consisting of alum and complete Freund's adjuvant.

**3.** A process according to claim 1 in which for each of R' and R" in the conjugate an alkyl group containing 15 carbon atoms, for R''' a cysteine residue, and for the adjuvant alum are used.

**4.** A process according to claim 3 in which said phosphatidyl choline, cholesterol and lysophosphatidyl choline are mixed in the liposome composition in the proportions by weight of 16:2:1, respectively.

**5.** A process according to any of claims 1 to 4 in which as Peptide Fragment in the formula of said conjugate is used

-Ser-Leu-Lys-Met-Ala-Asp-Pro-
Asn-Arg-Phe-Arg-Gly-Lys-Asn-
Leu-Pro-.

**6.** A process according to claim 3 or 4 in which as Peptide Fragment in the formula of said conjugate is used

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro-.

**7.** A process according to any of claims 1 to 4 in which as Peptide Fragment in the formula of said conjugate is used

-Ser-Leu-Lys-Met-Ala-Asp-Pro -
Asn-Arg-Phe-Arg-Gly-Lys-
Asp-Leu-Pro-.

**8.** A process according to any of claims 1 to 4 in which as Peptide Fragment in the formula of said conjugate is used

-Lys-Tyr-Ala-Leu-Ala-Asp-Ala-
Ser-Leu-Lys-Met-Ala-Asp-Pro-Asn-
Arg-Phe-Arg-Gly-Lys-Asp-Leu-Pro-.

**9.** A process for the preparation of a peptide-fatty acid conjugate having the formula

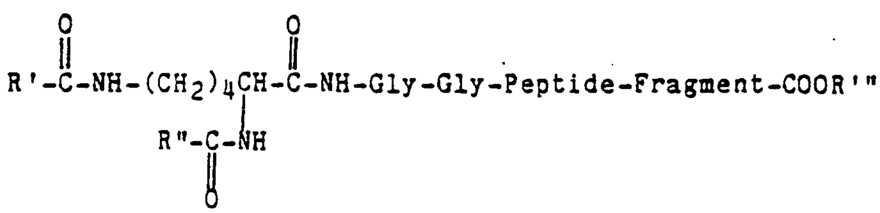

where R' and R'' are alkyl groups containing from 5 to 30 carbon atoms, R''' is selected from the group consisting of hydrogen and at least one amino acid residue, and said peptide fragment has an amino acid sequence corresponding to the Sequence of a fragment of herpes simplex virus type 1 envelope glycoprotein gD-1 or herpes simplex virus type 2 envelope glycoprotein gD-2 or a synthetic replica of said fragment, whereby said conjugate induces a T cell protective response in the substantial absence of an antibody response; in which process a spacer of Gly-Gly-Lys is added to an N-terminal region of a fragment of gD-1 or gD-2 which induces a T cell response or to a synthetic replica of such a fragment, and thus fatty acid side chains are coupled to the N-terminal lysine residue by the symmetric anhydride method.

**10.** A process according to claim 9 in which each of R' and R'' is an alkyl group containing from 10 to 20 carbon atoms, and R''' is a cysteine residue.

**11.** A process according to claim 9 in which each of R' and R'' is an alkyl group containing 15 carbon atoms, and R''' is a cysteine residue.

**12.** A process according to any of claims 9 to 11 in which the Peptide Fragment in the formula is

-Ser-Leu-Lys-Met-Ala-Asp-Pro-
Asn-Arg-Phe-Arg-Gly-Lys-Asn-
Leu-Pro- .

**13.** A process according to claim 11 in which as Peptide Fragment in the formula is used

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro- .

**14.** A process according to any of claims 9 to 11 in which as Peptide Fragment in the formula is used

-Ser-Leu-Lys-Met-Ala-Asp-Pro-
Asn-Arg-Phe-Arg-Gly-Lys-
Asp-Leu-Pro- .

**15.** A process according to any of claims 9 to 11 in which as Peptide Fragment in the formula is used

Lys-Tyr-Ala-Leu-Ala-Asp-Ala-
Ser-Leu-Lys-Met-Ala-Asp-Pro-Asn-
Arg-Phe-Arg-Gly-Lys-Asp-Leu-Pro-.

**16.** A process for preparing a composition useful for forming a vaccine for generating a T cell response protective against a herpes simplex virus, type 1 or type 2, when combined with an adjuvant, said process comprising mixing of immunologically effective amounts of (1) a peptide-fatty acid conjugate prepared according to any of claims 9 to 15 in which said peptide fragment has an amino acid sequence corresponding to the sequence of a fragment of herpes simplex virus type 1 envelope glycoprotein gD-1 or herpes simplex virus type 2 envelope glycoprotein gD-2 or a synthetic replica of said fragment, and (2) a liposome composition comprising a mixture of phosphatidyl choline, cholesterol and lysophosphatidyl choline, said composition being capable of inducing a T cell protective response in the substantial absence of an antibody response.

**17.** A process according to claim 16 in which said phosphatidyl choline, cholesterol and lysophoshatidyl choline are mixed in the liposome composition in the proportions by weight of 16:2:1, respectively.

**18.** A process according to claim 16 or 17 in which as Peptide Fragment in the formula of said conjugate is used

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro-.

**Revendications**

**Revendications pour les Etats contractants suivants : BE, CH, DE, FR, GB, IT, LI, LU, NL, SE**

1. Vaccin produisant une réponse en cellules T immunogènes protectrice contre un virus de l'herpès simple de type 1 ou de type 2, ledit vaccin comportant une quantité efficace du point de vue immunologique (1) d'un conjugué de peptide-acide gras, ledit peptide ayant une séquence en acides aminés correspondant à la séquence d'un fragment de glycoprotéine d'enveloppe gD-1 du virus de l'herpès simple de type 1 ou de glycoprotéine d'enveloppe gD-2 du virus de l'herpès simple de type 2 ou d'une réplique synthétique dudit fragment, ledit conjugé répondant à la formule

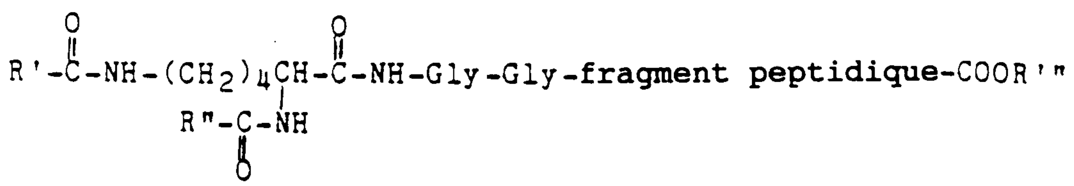

où R' et R'' sont des groupements alkyle renfermant de 5 à 30 atomes de carbone, et R''' est choisi dans le groupe constitué d'hydrogène et d'au moins un résidu d'acide aminé; (2) d'une composition de liposome comportant un mélange de phosphatidyle choline, de cholestérol et de lysophosphatidyle choline, et (3) d'un adjuvant, au moyen desquels ledit vaccin induit une réponse en cellules T protectrice dans l'absence substantielle d'une réponse en anticorps.

2. Vaccin selon la revendication 1 caractérisé en ce que R' et R'' sont chacun un groupement alkyle renfermant de 10 à 20 atomes de carbone, R''' est un résidu de cystéine, et l'adjuvant est choisi dans le groupe constitué de l'alun et de l'adjuvant complet de Freund.

3. Vaccin selon la revendication 1 caractérisé en ce que R' et R'' sont chacun un groupement alkyle renfermant 15 atomes de carbone, R''' est un résidu de cystéine, et l'adjuvant est l'alun.

4. Vaccin selon la revendication 3, caractérisé en ce que lesdits phosphatidyle choline, cholestérol et lysophosphatidyle choline sont présents dans les proportions en poids de 16:2:1 respectivement.

5. Vaccin selon l'une quelconque des revendications 1 à 4, caractérisé en ce que le fragment peptidique dans la formule dudit conjugué est

-Ser-Leu-Lys-Met-Ala-Asp-Pro-
Asn-Arg-Phe-Arg-Gly-Lys-Asn-
Leu-Pro-.

6. Vaccin selon la revendication 3 ou 4, caractérisé en ce que le fragment peptidique dans la formule dudit conjugué est

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro-.

**7.** Vaccin selon l'une quelconque des revendications 1 à 4, caractérisé en ce que le fragment peptidique dans la formule dudit conjugué est

-Ser-Leu-Lys-Met-Ala-Asp-Pro -
Asn-Arg-Phe-Arg-Gly-Lys-
Asp-Leu-Pro-.

**8.** Vaccin selon l'une quelconque des revendications 1 à 4, caractérisé en ce que le fragment peptidique dans la formule dudit conjugué est

-Lys-Tyr-Ala-Leu-Ala-Asp-Ala-
Ser-Leu-Lys-Met-Ala-Asp-Pro-Asn-
Arg-Phe-Arg-Gly-Lys-Asp-Leu-Pro-.

**9.** Conjugué de peptide-acide gras répondant à la formule

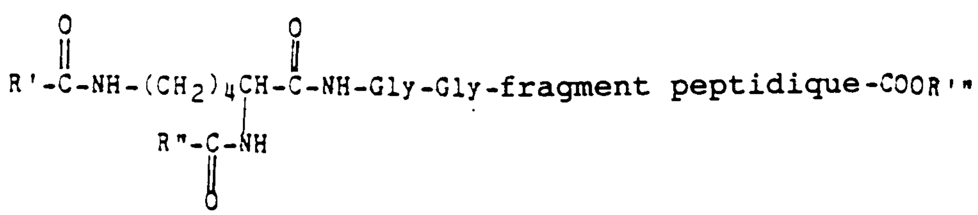

où R' et R'' sont des groupements alkyle renfermant de 5 à 30 atomes de carbone, R''' est choisi dans le groupe constitué d'hydrogène et d'au moins un résidu d'acide aminé, et ledit fragment peptidique a une séquence en acides aminés correspondant à la séquence d'un fragment de glycoprotéine d'enveloppe gD-1 du virus de l'herpès simple de type 1 ou de glycoprotéine d'enveloppe gD-2 du virus de l'herpès simple de type 2 ou d'une réplique synthétique dudit fragment, au moyen desquels ledit conjugué induit une réponse en cellules T protectrice dans l'absence substantielle d'une réponse en anticorps.

**10.** Conjugué selon la revendication 9, caractérisé en ce que R' et R'' sont chacun un groupement alkyle renfermant de 10 à 20 atomes de carbone, et R''' est un résidu de cystéine.

**11.** Conjugué selon la revendication 9, caractérisé en ce que R' et R'' sont chacun un groupement alkyle renfermant 15 atomes de carbone, et R''' est un résidu de cystéine.

**12.** Conjugué selon l'une quelconque des revendications 9 à 11, caractérisé en ce que le fragment peptidique dans la formule est

-Ser-Leu-Lys-Met-Ala-Asp-Pro-
Asn-Arg-Phe-Arg-Gly-Lys-Asn-
Leu-Pro- .

13. Conjugué selon la revendication 11, caractérisé en ce que le fragment peptidique dans la formule est

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro- .

14. Conjugué selon l'une quelconque des revendications 9 à 11, caractérisé en ce que le fragment peptidique dans la formule est

-Ser-Leu-Lys-Met-Ala-Asp-Pro-
Asn-Arg-Phe-Arg-Gly-Lys-
Asp-Leu-Pro- .

15. Conjugué selon l'une quelconque des revendications 9 à 11, caractérisé en ce que le fragment peptidique dans la formule est

Lys-Tyr-Ala-Leu-Ala-Asp-Ala-
Ser-Leu-Lys-Met-Ala-Asp-Pro-Asn-
Arg-Phe-Arg-Gly-Lys-Asp-Leu-Pro- .

16. Composition utile pour former un vaccin produisant une réponse en cellules T protectrice contre un virus de l'herpès simple de type 1 ou de type 2 lorsqu'elle est associée à un adjuvant, comportant une quantité efficace du point de vue immunologique (1) d'un conjugué de peptide-acide gras selon l'une quelconque des revendications 9 à 15 dans lequel ledit fragment peptidique a une séquence en acides aminés correspondant à la séquence d'un fragment de glycoprotéine d'enveloppe gD-1 du virus de l'herpès simple de type 1 ou de glycoprotéine d'enveloppe gD-2 du virus de l'herpès simple de type 2 ou d'une réplique synthétique dudit fragment, et (2) d'une composition de liposome comportant un mélange de phosphatidyle choline, de cholestérol et de lysophosphatidyle choline, ladite composition pouvant induire une réponse en cellules T protectrice dans l'absence substantielle d'une réponse en anticorps.

17. Composition selon la revendication 16, caractérisée en ce que lesdits phosphatidyle choline, cholestérol et lysophosphatidyle choline sont présents dans les proportions en poids de 16:2:1 respectivement.

18. Composition selon la revendication 16 ou 17, caractérisée en ce que le fragment peptidique dans la formule dudit conjugué est

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro-.

**Revendications pour l'Etat contractant suivant : AT**

1. Procédé de préparation d'un vaccin produisant une réponse en cellules T immunogènes protectrice contre un virus de l'herpès simple de type 1 ou de type 2, ledit procédé comportant le mélange de quantités efficaces du point de vue immunologique (1) d'un conjugué de peptide-acide gras, ledit peptide ayant une séquence en acides aminés correspondant à la séquence d'un fragment de glycoprotéine d'enveloppe gD-1 du virus de l'herpès simple de type 1 ou de glycoprotéine d'enveloppe gD-2 du virus de l'herpès simple de type 2 ou d'une réplique synthétique dudit fragment, ledit conjugué répondant à la formule

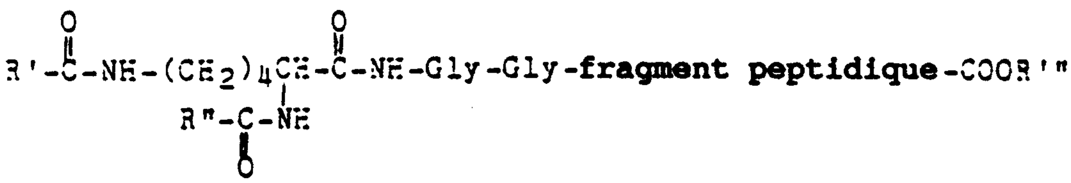

où R' et R'' sont des groupements alkyles renfermant de 5 à 30 atomes de carbone, et R''' est choisi dans le groupe constitué d'hydrogène et d'au moins un résidu d'acide aminé; (2) d'une composition de liposome comportant un mélange de phosphatidyle choline, de cholestérol et de lysophosphatidyle choline, et (3) d'un adjuvant, de telle sorte que le vaccin préparé induise une réponse en cellules T protectrice dans l'absence substantielle d'une réponse en anticorps.

2. Procédé selon la revendication 1, caractérisé en ce que pour chaque R' et R'' dans le conjugué, un groupement alkyle renfermant de 10 à 20 atomes de carbone, et pour R''', un résidu de cystéine, sont utilisés, et l'adjuvant est choisi dans le groupe constitué de l'alun et de l'adjuvant complet de Freund.

3. Procédé selon la revendication 1, caractérisé en ce que pour chaque R' et R'' dans le conjugué, un groupement alkyle renfermant 15 atomes de carbone, pour R''', un résidu de cystéine, et pour l'adjuvant, l'alun, sont utilisés.

4. Procédé selon la revendication 3, caractérisé en ce que lesdits phosphatidyle choline, cholestérol et lysophosphatidyle choline sont mélangés dans la composition de liposome dans les proportions en poids de 16:2:1 respectivement.

5. Procédé selon l'une quelconque des revendications 1 à 4, caractérisé en ce qu'à titre de fragment peptidique dans la formule dudit conjugué, on utilise

-Ser-Leu-Lys-Met-Ala-Asp-Pro-
Asn-Arg-Phe-Arg-Gly-Lys-Asn-
Leu-Pro-.

6. Procédé selon la revendication 3 ou 4, caractérisé en ce qu'à titre de fragment peptidique dans la formule dudit conjugué, on utilise

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro-.

7. Procédé selon l'une quelconque des revendications 1 à 4, caractérisé en ce qu'à titre de fragment peptidique dans la formule dudit conjugué, on utilise

-Ser-Leu-Lys-Met-Ala-Asp-Pro-
Asn-Arg-Phe-Arg-Gly-Lys-
Asp-Leu-Pro-.

8. Procédé selon l'une quelconque des revendications 1 à 4, caractérisé en ce qu'à titre de fragment peptidique dans la formule dudit conjugué, on utilise

-Lys-Tyr-Ala-Leu-Ala-Asp-Ala-
Ser-Leu-Lys-Met-Ala-Asp-Pro-Asn-
Arg-Phe-Arg-Gly-Lys-Asp-Leu-Pro-.

9. Procédé de préparation d'un conjugué de peptide-acide gras répondant à la formule

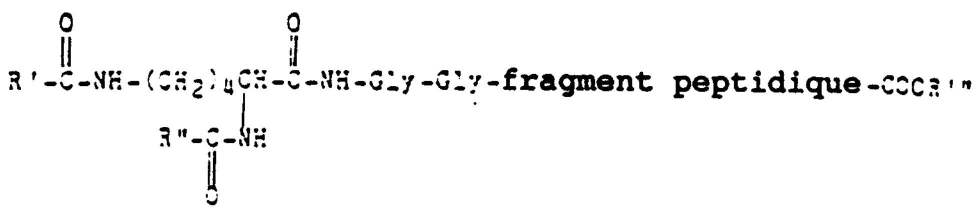

où R' et R'' sont des groupements alkyles renfermant de 5 à 30 atomes de carbone, R''' est choisi dans le groupe constitué d'hydrogène et d'au moins un résidu d'acide aminé, et ledit fragment peptidique a une séquence en acides aminés correspondant à la séquence d'un fragment de glycoprotéine d'enveloppe gD-1 du virus de l'herpès simple de type 1 ou de glycoprotéine d'enveloppe gD-2 du virus de l'herpès simple du type 2 ou d'une réplique synthétique dudit fragment, au moyen desquels ledit conjugué induit une réponse en cellules T protectrice dans l'absence substantielle d'une réponse en anticorps; dans lequel procédé un motif d'écartement de Gly-Gly-Lys est ajouté à une région N-terminale d'un fragment de gD-1 ou gD-2 qui induit une réponse en cellules T ou à une réplique synthétique d'un tel fragment, et deux chaînes latérales d'acides gras sont couplées au résidu de lysine de l'extrémité N-terminale par la méthode d'anhydride symétrique.

10. Procédé selon la revendication 9, caractérisé en ce que R' et R'' sont chacun un groupement alkyle renfermant de 10 à 20 atomes de carbone, et R''' est un résidu de cystéine.

11. Procédé selon la revendication 9, caractérisé en ce que R' et R'' sont chacun un groupement alkyle renfermant 15 atomes de carbone, et R''' est un résidu de cystéine.

12. Procédé selon l'une quelconque des revendications 9 à 11, caractérisé en ce qu'à titre de fragment peptidique dans la formule, on utilise

-Ser-Leu-Lys-Met-Ala-Asp-Pro-
Asn-Arg-Phe-Arg-Gly-Lys-Asn-
Leu-Pro- .

13. Procédé selon la revendication 11, caractérisé en ce qu'à titre de fragment peptidique dans la formule, on utilise

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro- .

14. Procédé selon l'une quelconque des revendications 9 à 11, caractérisé en ce qu'à titre de fragment peptidique dans la formule, on utilise

-Ser-Leu-Lys-Met-Ala-Asp-Pro-
Asn-Arg-Phe-Arg-Gly-Lys-
Asp-Leu-Pro- .

15. Procédé selon l'une quelconque des revendications 9 à 11, caractérisé en ce qu'à titre de fragment peptidique dans la formule, on utilise

Lys-Tyr-Ala-Leu-Ala-Asp-Ala-
Ser-Leu-Lys-Met-Ala-Asp-Pro-Asn-
Arg-Phe-Arg-Gly-Lys-Asp-Leu-Pro

16. Procédé de préparation d'une composition utile pour former un vaccin produisant une réponse en cellules T protectrice contre un virus de l'herpès simple de type 1 ou de type 2 lorsqu'elle est associée à un adjuvant, ledit procédé comportant le mélange de quantités efficaces du point de vue immunologique (1) d'un conjugué de peptide-acide gras préparé selon l'une quelconque des revendications 9 à 15 dans lequel ledit fragment peptidique a une séquence en acides aminés correspondant à la séquence d'un fragment de glycoprotéine d'enveloppe gD-1 du virus de l'herpès simple de type 1 ou de glycoprotéine d'enveloppe gD-2 du virus de l'herpès simple de type 2 ou d'une réplique synthétique dudit fragment, et (2) d'une composition de liposome comportant un mélange de phosphatidyle choline, de cholestérol et de lysophosphatidyle choline, ladite composition pouvant induire une réponse en cellules T protectrice dans l'absence substantielle d'une réponse en anticorps.

17. Procédé selon la revendication 16, caractérisé en ce que lesdits phosphatidyle choline, cholestérol et lysophosphatidyle choline sont mélangés dans la composition de liposome dans les proportions en poids de 16:2:1 respectivement.

18. Procédé selon la revendication 16 ou 17, caractérisé en ce qu'à titre de fragment peptidique dans la

formule dudit conjugué, on utilise

-Lys-Tyr-Ala-Leu-Ala-Asp-Pro-
Ser-Leu-Lys-Met-Ala-Asp-Pro-
Asn-Arg-Phe-Arg-Gly-Lys-Asn-
Leu-Pro-.

**Patentansprüche**

**Patentansprüche für folgende Vertragsstaaten : BE, CH, DE, FR, GB, IT, LI, LU, NL, SE**

**1.** Impfstoff, der eine immunogene, gegen den Herpes-simplex-Virus vom Typ 1 oder Typ 2 schützende T-Zellenreaktion hervorruft, wobei jener Impfstoff eine immunologisch wirksame Menge (1) eines Konjugats aus Peptid und Fettsäure, wobei jenes Peptid eine der Sequenz eines Fragments des Herpes-simplex-Virus vom Typ 1 mit einer Hülle aus Glykoprotein gD-1 oder des Herpes-simplex-Virus vom Typ 2 mit einer Hülle aus Glykoprotein gD-2 oder einer synthetischen Nachbildung jenes Fragments entsprechenden Aminosäuresequenz aufweist, wobei jenes Konjugat die Formel

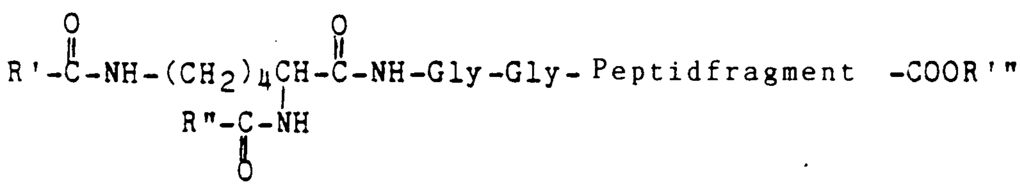

worin R' und R'' Alkylgruppen mit 5 bis 30 Kohlenstoffatomen bedeuten und R''' aus der Gruppe bestehend aus Wasserstoff und mindestens einem Aminosäurerest ausgewählt ist; (2) eine Liposomzusammensetzung bestehend aus einer Mischung aus Phosphatidylcholin, Cholesterin und Lysophosphatidylcholin und (3) eines Adjuvans, wodurch jener Impfstoff bei weitgehender Abwesenheit einer Antikörperreaktion eine schützende T-Zellenreaktion hervorruft, enthält.

**2.** Impfstoff nach Anspruch 1, worin R' und R'' jeweils eine Alkylgruppe mit 10 bis 20 Kohlenstoffatomen und R''' einen Zysteinrest bedeuten, und das Adjuvans aus der Gruppe bestehend aus Alaun und komplettem Adjuvans nach Freund ausgewählt ist.

**3.** Impfstoff nach Anspruch 1, worin R' und R'' jeweils eine Alkylgruppe mit 15 Kohlenstoffatomen und R''' einen Zysteinrest bedeuten, und das Adjuvans Alaun ist.

**4.** Impfstoff nach Anspruch 3, worin jenes Phosphatidylcholin, Cholesterin und Lysophosphatidylcholin in einem Gewichtsverhältnis von jeweils 16:2:1 enthalten sind.

**5.** Impfstoff nach einem der Ansprüche 1 bis 4, worin es sich bei dem Peptidfragment in der Formel jenes Konjugats um

-Ser-Leu-Lys-Met-Ala-Asp-Pro-Asn-Arg-Phe-Arg-Gly-
Lys-Asn-Leu-Pro-

handelt.

**6.** Impfstoff nach Anspruch 3 oder 4, worin es sich bei dem Peptidfragment in der Formel jenes Konjugats um

-Lys-Tyr-Ala-Leu-Ala-Asp-Pro-Ser-Leu-Lys-Met-Ala-Asp-Pro-Asn-Arg-Phe-Arg-Gly-Lys-Asn-Leu-Pro-

handelt.

**7.** Impfstoff nach einem der Ansprüche 1 bis 4, worin es sich bei dem Peptidfragment in der Formel jenes Konjugats um

-Ser-Leu-Lys-Met-Ala-Asp-Pro-Asn-Arg-Phe-Arg-Gly-Lys-Asp-Leu-Pro-

handelt.

**8.** Impfstoff nach einem der Ansprüche 1 bis 4, worin es sich bei dem Peptidfragment in der Formel jenes Konjugats um

-Lys-Tyr-Ala-Leu-Ala-Asp-Ala-Ser-Leu-Lys-Met-Ala-Asp-Pro-Asn-Arg-Phe-Arg-Gly-Lys-Asp-Leu-Pro-

handelt.

**9.** Konjugat aus Peptid und Fettsäure mit der Formel

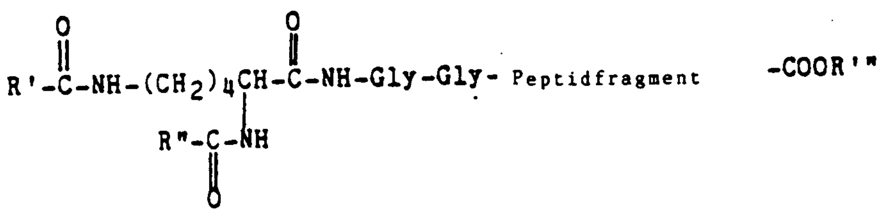

worin R' und R'' Alkylgruppen mit 5 bis 30 Kohlenstoffatomen bedeuten, R''' aus der Gruppe bestehend aus Wasserstoff und mindestens einem Aminosäurerest ausgewählt ist und jenes Peptidfragment eine der Sequenz eines Fragments des Herpes-simplex-Virus vom Typ 1 mit einer Hülle aus Glykoprotein gD-1 oder des Herpes-simplex-Virus vom Typ 2 mit einer Hülle aus Glykoprotein gD-2 oder einer synthetischen Nachbildung jenes Fragments entsprechende Aminosäuresequenz aufweist, wodurch jenes Konjugat bei weitgehender Abwesenheit einer Antikörperreaktion eine schützende T-Zellenreaktion auslöst.

**10.** Konjugat nach Anspruch 9, worin R' und R'' jeweils eine Alkylgruppe mit 10 bis 20 Kohlenstoffatomen und R''' einen Zysteinrest bedeuten.

**11.** Konjugat nach Anspruch 9, worin R' und R'' jeweils eine Alkylgruppe mit 15 Kohlenstoffatomen und R''' einen Zysteinrest bedeuten.

**12.** Konjugat nach einem der Ansprüche 9 bis 11, worin es sich bei dem Peptidfragment in der Formel um

-Ser-Leu-Lys-Met-Ala-Asp-Pro-Asn-Arg-Phe-Arg-Gly-Lys-Asn-Leu-Pro-

handelt.

**13.** Konjugat nach Anspruch 11, worin es sich bei dem Peptidfragment in der Formel um

**-Lys-Tyr-Ala-Leu-Ala-Asp-Pro-Ser-Leu-Lys-Met-Ala-Asp-Pro-Asn-Arg-Phe-Arg-Gly-Lys-Asn-Leu-Pro-**

handelt.

**14.** Konjugat nach einem der Ansprüche 9 bis 11, worin es sich bei dem Peptidfragment in der Formel um

**-Ser-Leu-Lys-Met-Ala-Asp-Pro-Asn-Arg-Phe-Arg-Gly-Lys-Asp-Leu-Pro-**

handelt.

**15.** Konjugat nach einem der Ansprüche 9 bis 11, worin es sich bei dem Peptidfragment in der Formel um

**-Lys-Tyr-Ala-Leu-Ala-Asp-Ala-Ser-Leu-Lys-Met-Ala-Asp-Pro-Asn-Arg-Phe-Arg-Gly-Lys-Asp-Leu-Pro-**

handelt.

**16.** Zusammensetzung, die sich zur Bildung eines Impfstoffes eignet, der eine gegen ein Herpes-simplex-Virus vom Typ 1 oder Typ 2 schützende T-Zellenreaktion bei Kombination mit einem Adjuvans hervorruft, und eine immunologisch wirksame Menge (1) eines Konjugats aus Peptid und Fettsäure nach einem der Ansprüche 9 bis 15, worin jenes Peptidfragment eine der Sequenz eines Fragments des Herpes-simplex-Virus vom Typ 1 mit einer Hülle aus Glykoprotein gD-1 oder des Herpes-simplex-Virus vom Typ 2 mit einer Hülle aus Glykoprotein gD-2 oder einer synthetischen Nachbildung jenes Fragments entsprechende Aminosäuresequenz aufweist, und (2) einer Liposomzusammensetzung bestehend aus einer Mischung aus Phosphatidylcholin, Cholesterin und Lysophosphatidylcholin enthält, wobei jene Zusammensetzung in der Lage ist, bei weitgehender Abwesenheit einer Antikörperreaktion eine schützende T-Zellenreaktion auszulösen.

**17.** Zusammensetzung nach Anspruch 16, worin jenes Phosphatidylcholin, Cholesterin und Lysophosphatidylcholin in einem Gewichtsverhältnis von jeweils 16:2:1 enthalten sind.

**18.** Zusammensetzung nach Anspruch 16 oder 17, worin es sich bei dem Peptidfragment in der Formel jenes Konjugats um

**-Lys-Tyr-Ala-Leu-Ala-Asp-Pro-Ser-Leu-Lys-Met-Ala-Asp-Pro-Asn-Arg-Phe-Arg-Gly-Lys-Asn-Leu-Pro-**

handelt.

**Patentansprüche für folgenden Vertragsstaat : AT**

**1.** Verfahren zur Herstellung eines Impfstoffes, der eine immunogene, gegen den Herpes-simplex-Virus vom Typ 1 oder Typ 2 schützende T-Zellenreaktion hervorruft, wobei jener Impfstoff eine immunologisch wirksame Menge (1) eines Konjugats aus Peptid und Fettsäure, wobei jenes Peptid eine der

Sequenz eines Fragments des Herpes-simplex-Virus vom Typ 1 mit einer Hülle aus Glykoprotein gD-1 oder des Herpes-simplex-Virus vom Typ 2 mit einer Hülle aus Glykoprotein gD-2 oder einer synthetischen Nachbildung jenes Fragments entsprechenden Aminosäuresequenz aufweist, wobei jenes Konjugat die Formel

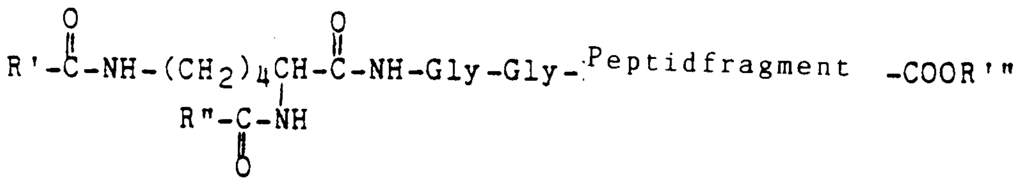

worin R' und R" Alkylgruppen mit 5 bis 30 Kohlenstoffatomen bedeuten und R''' aus der Gruppe bestehend aus Wasserstoff und mindestens einem Aminosäurerest ausgewählt ist; (2) einer Liposomzusammensetzung bestehend aus einer Mischung aus Phosphatidylcholin, Cholesterin und Lysophosphatidylcholin und (3) eines Adjuvans, so daß der hergestellte Impfstoff bei weitgehender Abwesenheit einer Antikörperreaktion eine schützende T-Zellenreaktion hervorruft, enthält.

**2.** Verfahren nach Anspruch 1, worin als R' und R" in dem Konjugat jeweils eine Alkylgruppe mit 10 bis 20 Kohlenstoffatomen und als R''' ein Zysteinrest verwendet werden, und das Adjuvans aus der Gruppe bestehend aus Alaun und komplettem Adjuvans nach Freund ausgewählt ist.

**3.** Verfahren nach Anspruch 1, worin als R' und R" in dem Konjugat jeweils eine Alkylgruppe mit 15 Kohlenstoffatomen, als R''' ein Zysteinrest, und als Adjuvans Alaun verwendet werden.

**4.** Verfahren nach Anspruch 3, worin jenes Phosphatidylcholin, Cholesterin und Lysophosphatidylcholin in der Liposomzusammensetzung in einem Gewichtsverhältnis von jeweils 16:2:1 gemischt werden.

**5.** Verfahren nach einem der Ansprüche 1 bis 4, worin als Peptidfragment in der Formel jenes Konjugats

**-Ser-Leu-Lys-Met-Ala-Asp-Pro-Asn-Arg-Phe-Arg-Gly-**
**Lys-Asn-Leu-Pro-**

verwendet wird.

**6.** Verfahren nach Anspruch 3 oder 4, worin als Peptidfragment in der Formel jenes Konjugats

**-Lys-Tyr-Ala-Leu-Ala-Asp-Pro-Ser-Leu-Lys-Met-Ala-**
**Asp-Pro-Asn-Arg-Phe-Arg-Gly-Lys-Asn-Leu-Pro-**

verwendet wird.

**7.** Verfahren nach einem der Ansprüche 1 bis 4, worin als Peptidfragment in der Formel jenes Konjugats

**-Ser-Leu-Lys-Met-Ala-Asp-Pro-Asn-Arg-Phe-Arg-Gly-**
**Lys-Asp-Leu-Pro-**

verwendet wird.

**8.** Verfahren nach einem der Ansprüche 1 bis 4, worin als Peptidfragment in der Formel jenes Konjugats

-Lys-Tyr-Ala-Leu-Ala-Asp-Ala-Ser-Leu-Lys-Met-Ala-
Asp-Pro-Asn-Arg-Phe-Arg-Gly-Lys-Asp-Leu-Pro-

verwendet wird.

**9.** Verfahren zur Herstellung eines Konjugats aus Peptid und Fettsäure mit der Formel

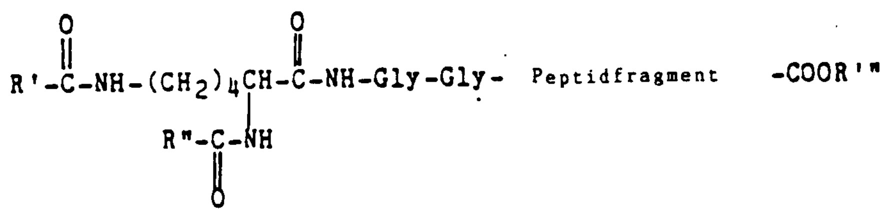

worin R' und R" Alkylgruppen mit 5 bis 30 Kohlenstoffatomen bedeuten, R''' aus der Gruppe bestehend aus Wasserstoff und mindestens einem Aminosäurerest ausgewählt ist und jenes Peptidfragment eine der Sequenz eines Fragments des Herpes-simplex-Virus vom Typ 1 mit einer Hülle aus Glykoprotein gD-1 oder des Herpes-simplex-Virus vom Typ 2 mit einer Hülle aus Glycoprotein gD-2 oder einer synthetischen Nachbildung jenes Fragments entsprechende Aminosäuresequenz aufweist, wodurch jenes Konjugat bei weitgehender Abwesenheit einer Antikörperreaktion eine schützende T-Zellenreaktion auslöst.

**10.** Verfahren nach Anspruch 9, worin R' und R" jeweils eine Alkylgruppe mit 10 bis 20 Kohlenstoffatomen und R''' einen Zysteinrest bedeuten.

**11.** Verfahren nach Anspruch 9, worin R' und R" jeweils eine Alkylgruppe mit 15 Kohlenstoffatomen und R''' einen Zysteinrest bedeuten, und bei dem ein Spacer aus Gly-Gly-Lys an den N-endständigen Bezirk eines Fragments aus gD-1 oder gD-2, wodurch eine T-Zellenreaktion ausgelöst wird, oder an die synthetische Nachbildung eines solchen Fragments addiert wird, und zwei Fettsäureseitenketten mittels der symmetrischen Anhydridmethode mit dem N-endständigen Lysinrest verkuppelt werden.

**12.** Verfahren nach einem der Ansprüche 9 bis 11, worin als Peptidfragment in der Formel

-Ser-Leu-Lys-Met-Ala-Asp-Pro-Asn-Arg-Phe-Arg-Gly-
Lys-Asn-Leu-Pro-

verwendet wird.

**13.** Verfahren nach Anspruch 11, worin als Peptidfragment in der Formel

-Lys-Tyr-Ala-Leu-Ala-Asp-Pro-Ser-Leu-Lys-Met-Ala-
Asp-Pro-Asn-Arg-Phe-Arg-Gly-Lys-Asn-Leu-Pro-

verwendet wird.

**14.** Verfahren nach einem der Ansprüche 9 bis 11, worin als Peptidfragment in der Formel

**-Ser-Leu-Lys-Met-Ala-Asp-Pro-Asn-Arg-Phe-Arg-Gly-Lys-Asn-Leu-Pro-**

verwendet wird.

**15.** Verfahren nach einem der Ansprüche 9 bis 11, worin als Peptidfragment in der Formel

**-Lys-Tyr-Ala-Leu-Ala-Asp-Ala-Ser-Leu-Lys-Met-Ala-Asp-Pro-Asn-Arg-Phe-Arg-Gly-Lys-Asp-Leu-Pro-**

verwendet wird.

**16.** Verfahren zur Herstellung einer Zusammensetzung, die sich zur Bildung eines Impfstoffes eignet, der eine gegen ein Herpes-simplex-Virus vom Typ 1 oder Typ 2 schützende T-Zellenreaktion bei Kombination mit einem Adjuvans hervorruft, wobei dieses Verfahren darin besteht, daß man immunologisch wirksame Mengen (1) eines nach einem der Ansprüche 9 bis 15 hergestellten Konjugats aus Peptid und Fettsäure, worin jenes Peptidfragment eine der Sequenz eines Fragments des Herpes-simplex-Virus vom Typ 1 mit einer Hülle aus Glykoprotein gD-1 oder des Herpes-simplex-Virus vom Typ 2 mit einer Hülle aus Glykoprotein gD-2 oder einer synthetischen Nachbildung jenes Fragments entsprechende Aminosäuresequenz aufweist, und (2) einer Liposomzusammensetzung bestehend aus einer Mischung aus Phosphatidylcholin, Cholesterin und Lysophosphatidylcholin vermischt, wobei jene Zusammensetzung in der Lage ist, bei weitgehender Abwesenheit einer Antikörperreaktion eine schützende T-Zellenreaktion auszulösen.

**17.** Verfahren nach Anspruch 16, worin jenes Phosphatidylcholin, Cholesterin und Lysophosphatidylcholin in der Liposomzusammensetzung in einem Gewichtsverhältnis von jeweils 16:2:1 vermischt werden.

**18.** Verfahren nach Anspruch 16 oder 17, worin als Peptidfragment in der Formel jenes Konjugats

**-Lys-Tyr-Ala-Leu-Ala-Asp-Pro-Ser-Leu-Lys-Met-Ala-Asp-Pro-Asn-Arg-Phe-Arg-Gly-Lys-Asn-Leu-Pro-**

verwendet wird.

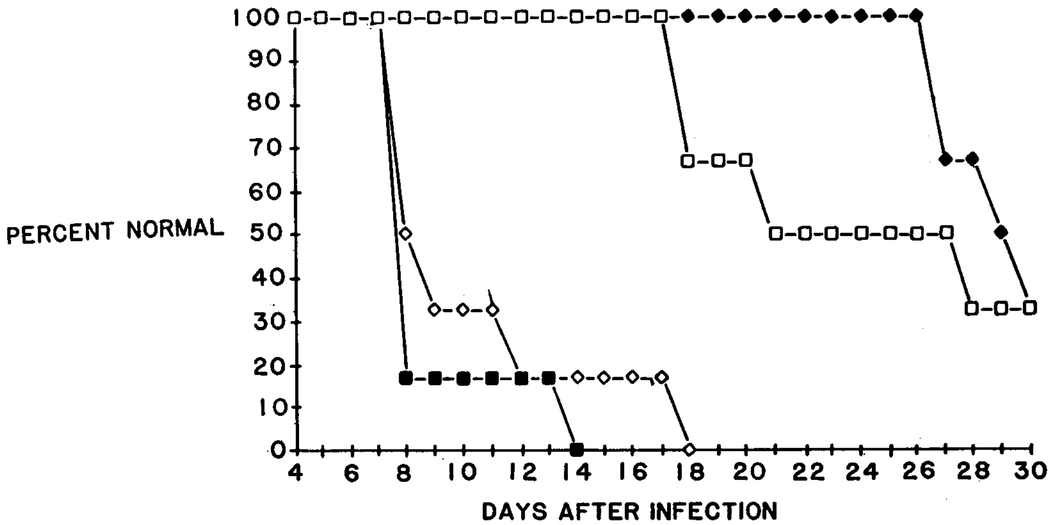
100
90
80
70
60
PERCENT NORMAL
50
40
30
20
10
0
4
6
8
10
12
14
16
18
20
22
24
26
28
30
DAYS AFTER INFECTION

FIG. 1

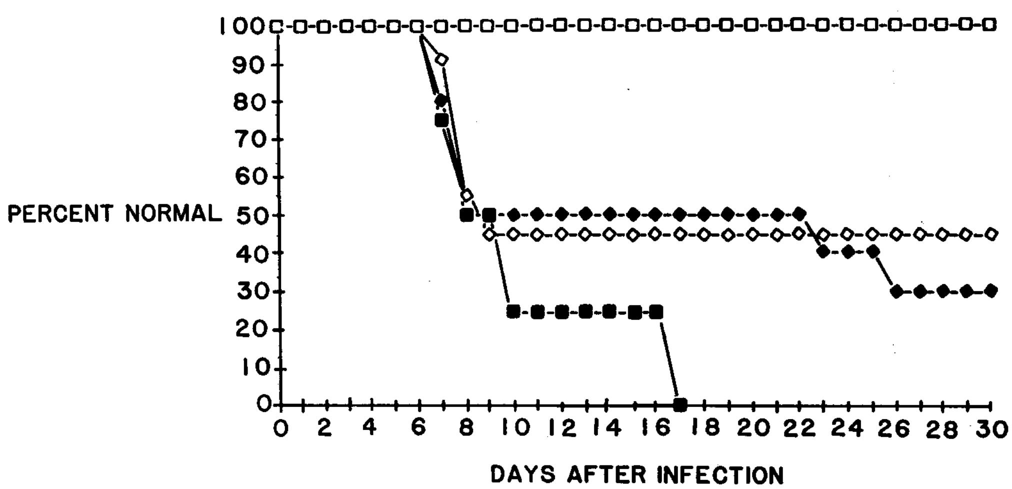
100
90
80
70
60
PERCENT NORMAL
50
40
30
20
10
0
0
2
4
6
8
10
12
14
16
18
20
22
24
26
28
30
DAYS AFTER INFECTION

FIG. 3

# FIG. 2A

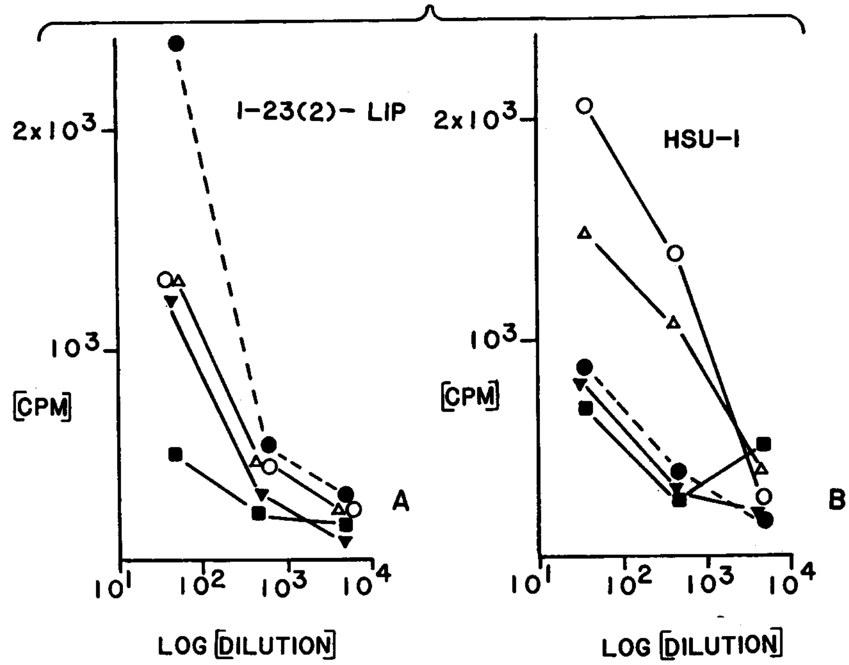
I-23(2)- LIP
2x10^3
10^3
[CPM]
10^1 10^2 10^3 10^4
LOG [DILUTION]
A
HSU-I
2x10^3
10^3
[CPM]
10^1 10^2 10^3 10^4
LOG [DILUTION]
B

# FIG. 2B

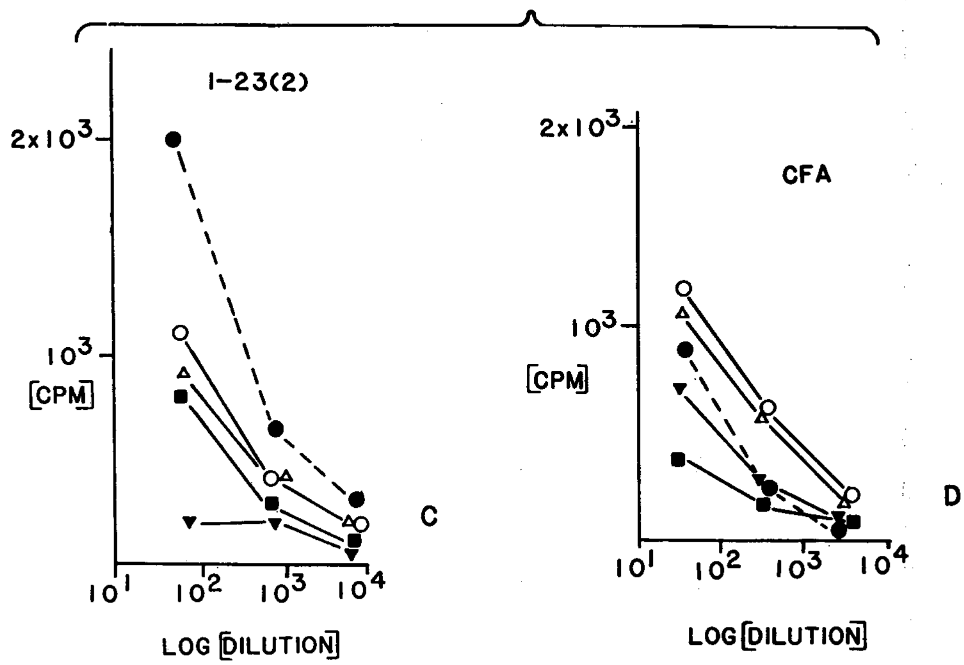
I-23(2)
2x10^3
10^3
[CPM]
10^1 10^2 10^3 10^4
LOG [DILUTION]
C
CFA
2x10^3
10^3
[CPM]
10^1 10^2 10^3 10^4
LOG [DILUTION]
D